# EUROPEAN PATENT APPLICATION

(11) **EP 3 806 102 A1**
(43) Date of publication of application: **14.04.2021**
(21) Application number: 19201622.8
(22) Date of filing: 07.10.2019
(51) Int. Cl.: G16H 40/20, G06Q 50/00, G06Q 10/06, A61B 5/00, A61B 5/021

(54) **A SYSTEM AND METHOD FOR SCHEDULING BLOOD PRESSURE MEASUREMENTS OF A SUBJECT**

(71) Applicant: Koninklijke Philips N.V., 5656 AG Eindhoven (NL)
(72) Inventor: LAMICHHANE, Bishal, 5656 AE Eindhoven (NL); MUEHLSTEFF, Jens, 5656 AE Eindhoven (NL)
(74) Representative: Philips Intellectual Property & Standards

(57) **Abstract**

A system and method for scheduling blood pressure measurements makes use of patient information relating to at least the activity of a patient and also timing information relating to an expected clinician visit, in order to determine a time for a next blood pressure measurement of the subject. In this way, the clinician is presented with an up-to-date blood pressure measurement and one which has been taken at a suitable activity level (e.g. at rest), also consistent with previous measurements.

## Description

### FIELD OF THE INVENTION

This invention relates to a system for scheduling blood pressure measurements of a subject, in particular in a hospital (or other medical care facility) setting, and in particular when a clinician carries out a rota of visits to the subject being monitored.

### BACKGROUND OF THE INVENTION

The monitoring of patients in a hospital setting is growing beyond just the monitoring of vital signs. As patient monitors are being designed with increasing functionality, they are developing into the role of a patient care console.

Beyond displaying vital signs and trends, these smart consoles use intelligence to aid clinical decision making and improve workflows. One aspect of a smart patient care console is the intelligent scheduling of vital signs monitoring in order to optimally assess a patient's medical status.

Blood pressure is one of the vital signs which can be intelligently scheduled to improve current clinical practice. Blood pressure is commonly measured to gain insight about a patient's cardiovascular status. The blood pressure value in conjunction with other vital signs like heart rate, respiration rate and temperature can be obtained by a clinician (doctor, nurse, or other trained caregiver) to understand a patient's current medical condition. This understanding is then used in deciding interventions and therapy for the patient, if necessary.

There are two main methods for blood pressure monitoring used in a clinical setting: arterial line based blood pressure measurement and oscillometry. Blood pressure measurement with an arterial line involves inserting a catheter with a pressure sensor into a patient's artery (usually the radial or femoral artery). It is an invasive measurement method and reserved for use in high acuity patients or during surgery. Such patients require continuous monitoring of blood pressure which is possible with an arterial line. However arterial line based blood pressure measurement also increases chances of infection and sepsis.

Therefore, a common clinical practice is to use arterial line based blood pressure measurement in only very sick patients and rather use oscillometry based blood pressure measurement for the majority of the patient population because of its non-invasiveness. For an oscillometric blood pressure measurement, a cuff wrapped around a limb (most of the time the upper arm) is inflated rapidly to certain high pressure. Then the cuff pressure is gradually or stepwise decreased. Blood pressure values are estimated from the amplitude of cuff pressure oscillations during deflation. This is a deflation based approach, but there are also inflation based approaches.

An oscillometric blood pressure measurement usually takes a few tens of seconds (around 20 seconds for inflation based blood pressure devices and 40 seconds for deflation based blood pressure devices). As such, only an intermittent snapshot of the blood pressure values can be obtained with such devices. This however is a reasonable tradeoff in a majority of the patient population considering the invasiveness and associated risks of an arterial line.

One standard practice, common mostly for patients in higher acuity monitoring, is that the blood pressure measurement device is always worn by the patient and connected to a bedside monitor. The blood pressure measurement device is then configured to trigger measurements intermittently. The interval of these measurements is decided by the clinician based on the perceived status of the patient. It is for example common to set a measurement interval of 5 minutes or less for very sick patients, about 15 minutes for sick patients, and 30 minutes or higher for relatively stable patients.

Short measurement intervals are only used when absolutely required as too frequent blood pressure measurements compromise the patient's comfort.

During a routine visit of a clinician to a patient in the bed, it is desirable to have a current snapshot of the measured vital signs. The current vital sign information along with the direct visual observation and interaction with the patient will give the best insight to the clinician regarding the patient's current status and well-being.

While vital signs like heart rate and respiration rate are measured on a continuous basis, blood pressure measurements are intermittent as mentioned above. It is therefore possible that the available blood pressure measurement on a patient monitor was taken quite some time before the clinician arrived in the bed. Long measurement intervals of 15 minutes or even 30 minutes are common in clinical practice to maximize patient comfort, and the patient might have an altered cardio-vascular status between the measurement instants. A blood pressure measurement can always be manually triggered by the clinician while they are visiting the patient bed, but this tends to provide a false reading due to the well-known white-coat effect. It also takes up additional clinician time.

There is therefore a need for an improved blood pressure scheduling approach.

### SUMMARY OF THE INVENTION

The invention is defined by the claims.

According to examples in accordance with an aspect of the invention, there is provided a system for scheduling blood pressure measurements of a subject, comprising:
a patient care module which comprises a blood pressure measurement system; and
a controller communicatively coupled to a patient context module arranged to generate patient information relating to recent patient activities and/or movements and coupled to a clinician module arranged to generate timing information relating to a next clinician visit, wherein
the controller is adapted to process the patient information and the timing information in order to determine a time for a next blood pressure measurement of the subject.

This system provides a blood pressure measurement schedule, which takes account of the patient context, including at least their recent activities and movements, and also the timing of an expected imminent clinician visit. In this way, it can be ensured that the blood pressure measurement is accurate and is not for example distorted by recent exertion of the subject, and also that when a clinician visits, the blood pressure measurement is very recent. Thus, it can correctly be associated with other indicators of the subject which are investigated by the clinician during their visit. The system preferably displays the time at which the currently provided blood pressure measurement was taken so that the clinician can judge if the measurement is relevant to the other investigations, or if another blood pressure measurement should be taken.

By scheduling a blood pressure measurement just before a clinician arrival, the so-called white coat effect is avoided, by which the presence of the clinician affects the results.

The system is preferably for a hospital bed setting, and is thus positioned at a hospital bed, and is used for subjects being monitored during patient rounds conducted by a clinician (doctor or nurse).

The controller is preferably further adapted to control the blood pressure measurement system to take a blood pressure measurement at the determined time of the next blood pressure measurement of the subject.. Thus, the system provides automatic scheduling and taking of blood pressure measurements. Of course, a manual override is possible, by which a blood pressure measurement may be taken at any time.

The blood pressure measurement system for example comprises a blood pressure measurement cuff. Since such blood pressure measurements cannot be taken continuously, a time schedule is needed.

The controller may be adapted, based on the received timing information relating to a next clinician visit, to implement periodic blood pressure measurements when there is no imminent clinician visit. Thus, the system performs a conventional blood pressure measurement schedule, for example every 15 minutes or every 30 minutes. However, even these regularly scheduled time points may be varied in dependence on the information from the patient context module, so that the blood pressure measurements may be taken with the subject at rest.

The controller may be adapted, based on the received timing information relating to a next clinician visit, to implement a blood pressure measurement within the 5 minutes preceding an expected clinician visit. Thus, the measurement is preferably before the clinician is present, but sufficiently late that the results are clearly representative of the current condition of the subject. There may be a target to take the measurement 5 minutes, 4 minutes, 3 minutes or 2 minutes before the clinician visit. Thus, a target timing may be set somewhere in the range 1 minute to 5 minutes before the clinician visit.

The system may further comprise the patient context module.

The patient context module may for example further comprise a set of one or more physiological sensors for monitoring physiological parameters of the subject, and the controller is further adapted to process the monitored physiological parameters to determine the time for a next blood pressure measurement of the subject.

Thus, in addition to physical activity or movement of the subject, various physiological measurements may be used to determine when a blood pressure measurement is most appropriate. The physiological sensors for example comprise one or more of a respiration rate sensor, a heart rate sensor and a temperature sensor.

The patient context module may for example be adapted to determine a heart rate variability. This may be used to provide an indication of stress of the subject, which may affect the accuracy of a blood pressure measurement.

The patient context module may for example generate patient information relating to one or more of:
recent nursing interventions;
medications taken by the subject; and
food consumption of the subject.

All of these contexts may influence when it is most appropriate to take a blood pressure measurement.

The patient context module for example comprises a camera system for tracking at least movement of the subject. A camera system may however identify many other factors, such as nurse interventions, feeding, etc. Wearable sensors may also or instead be used.

The system may further comprise the clinician module.

The clinician module for example comprises:
an input for receiving camera information for detecting the presence of the clinician at a location in advance of the location of the subject; and/or
an input for receiving location tracking information for tracking the location of the clinician.

These are two ways to determine how far around the rota of patient visits the clinician has reached, and hence when arrival at the subject being monitored is expected.

The clinician module for example receives patient schedule information for the clinician, wherein the patient schedule is dynamically adjusted based on the timing of previous patient visits by the clinician.

Thus, the clinician may have a schedule, which indicates the order and a default timing of patient visits. This schedule may then be adjusted based on the actual visit times, which depend on the variable times of the patient interactions, and any disruptions or interruptions, which the clinician may encounter.

The invention also provides a computer-implemented method for scheduling blood pressure measurements of a subject, comprising:
receiving patient information relating to recent patient activities and/or movements;
receiving timing information relating to a next clinician visit to the subject; and
processing the patient information and the timing information in order to determine a time for a next blood pressure measurement of the subject.

The method may further comprise monitoring physiological parameters of the subject, and also processing the monitored physiological parameters to determine the time for a next blood pressure measurement of the subject.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a hospital bed and a patient monitoring system at the bedside;
Figure 2 shows the system for scheduling blood pressure measurements of a subject on the hospital bed; and
Figure 3 shows a computer-implemented method for scheduling blood pressure measurements of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides system and method for scheduling blood pressure measurements that makes use of patient information relating to at least the activity of a patient and also timing information relating to an expected clinician visit, in order to determine a time for a next blood pressure measurement of the subject. In this way, the clinician is presented with an up-to-date blood pressure measurement and one which has been taken at a suitable activity level (e.g. at rest), also consistent with previous measurements.

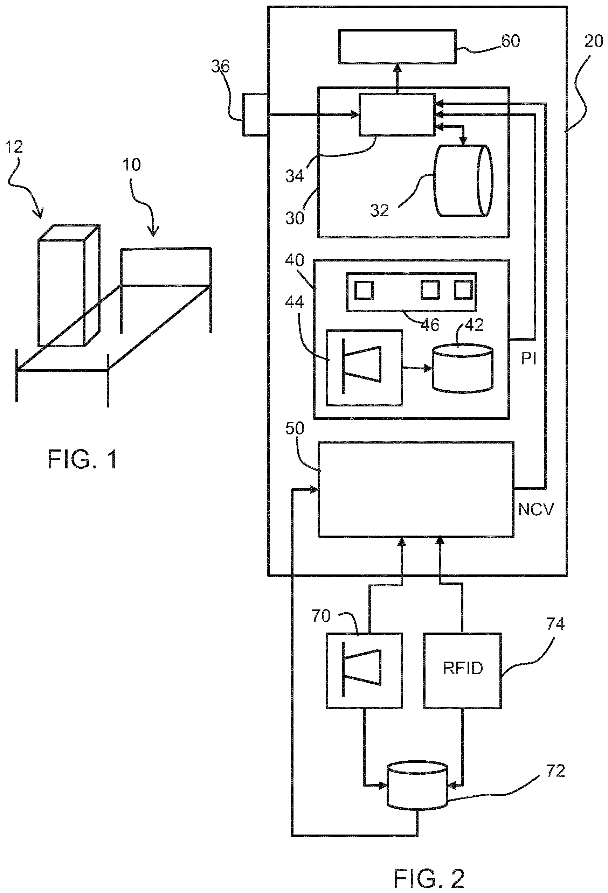

Figure 1 shows a hospital bed 10, or a bed in any other medical care facility, and a patient monitoring system 12 at the bedside.

The invention relates to the implementation of a system for scheduling blood pressure measurements, and preferably automatically performing those measurements, incorporated into the patient monitoring system 12.

Figure 2 shows the system 20 for scheduling the blood pressure measurements of a subject on the hospital bed.

The system 20 comprises a patient care module 30 which includes a blood pressure measurement system 32. The blood pressure measurement system 32 is a blood pressure measurement cuff for oscillometric blood pressure measurement. The system in a preferred example is for automatically performing the blood pressure measurements according to a determined schedule, under the control of a controller 34 (shown as part of the patient care module, but only by way of example). However, a manual override is possible by input 36, by which a blood pressure measurement may be taken at any time.

A patient context module 40 generates patient information "PI" relating at least to recent patient activities and/or movements. A memory 42 stores this information.

A clinician module 50 generates timing information relating to a next clinician visit "NCV".

A monitor, i.e. display, 60 provides a blood pressure output. It also displays the time at which the currently provided blood pressure measurement was taken so that a clinician can judge if the measurement is relevant to the other investigations, or if another blood pressure measurement should be taken, using the manual input 36.

The controller 34 of the patient care module 30 processes the patient information PI and the timing information NCV in order to determine a time for a next blood pressure measurement of the subject.

The blood pressure measurement system 32 is then controlled to perform the measurement. It is possible for the system instead simply to provide scheduling information, for example to trigger a bedside carer to initiate the measurement, but the system is preferably for automatically performing the blood pressure measurements.

The system is an extension of existing patient monitor systems, and some of the components described below are already present parts of a bedside patient monitor system.

It is noted that Figure 2 shows various parts of the system implemented at the bedside. However, the invention relates more generally to the processing of the information from the patient context module and the clinician module in order to schedule the blood pressure measurements. The patient context module and the clinician module may be located remotely and simply provide information to the controller (in a wired or wireless manner), which then controls the blood pressure measurement process. Thus, Figure 2 shows only one possible implementation. Other implementations may have a more distributed configuration.

The various components will now be described in more detail.

The patient context module 40 comprises a camera system 44 for tracking at least the movement of the subject. The camera system 44 may however identify many other factors, such as nurse interventions, medications taken, feeding, etc. These factors compromise blood pressure measurements in such a way that the measured values do not accurately reflect the baseline cardiovascular status. For example, the blood pressure might rise temporarily due to heavy movement and may return back to its baseline only after some period of delay.

Besides camera-based solutions, other information sources may be used such as the EMR (Electronic Medical Record) system to obtain information relating to medication or nursing interventions, or wearable sensors to derive patient movement and pose context. The patient context may also relate to their current stress level or sleep stages, derived from other vital sign information sources like ECG or EEG monitoring.

The patient context module 40 for example has a set 46 of (one or more) physiological sensors for monitoring physiological parameters of the subject. The patient information PI thus includes the monitored physiological parameters, and in turn the controller 34 determines the time for a next blood pressure measurement of the subject using this additional information. In this way, in addition to physical activity or movement of the subject detected by the camera system 44, various physiological measurements may be used to determine when a blood pressure measurement is most appropriate. The physiological sensors for example comprise one or more of a respiration rate sensor, a heart rate sensor and a (body) temperature sensor.

The system may (either at the controller 34 or within the module 40) for example determine a heart rate variability. This may be used to provide an indication of stress of the subject, which may affect the accuracy of a blood pressure measurement.

The patient context module 40 thus enables increased intelligence in the selection of the time at which a blood pressure measurement should be taken. A blood pressure measurement may be taken after a suitable delay following recent movement (i.e. physical activity) of the subject, which might influence the blood pressure measurement. A delay may similarly be implemented if a period of heightened stress is detected.

For example, even regularly scheduled time points for blood pressure measurement (outside of times when the clinician is about to visit) may be varied by the delays mentioned above, in dependence on the information from the patient context module.

The timing of blood pressure measurements is optimized so that when the clinician is at the bedside to visit and assess the patient, the blood pressure measurement displayed at the monitor 60 reflects a recent measurement and thus current status of the patient. This avoids that the blood pressure values displayed while the clinician is at the bedside may be old and not representative of the current status of the patient.

The purpose of the clinician module 50 is to obtain or generate information regarding an upcoming visit of a clinician as mentioned above. The patient care module 30 then schedules the triggering of a blood pressure measurement in accordance with the expected visit of the clinician, in addition to any other triggering scheme it may be operating e.g. a uniform triggering scheme which measuring periodically for example every 15 or minutes or every hour.

The blood pressure measurement is for example be timed so that it takes place within the 5 minutes preceding an expected clinician visit. Thus, the measurement is preferably before the clinician is present, but sufficiently late that the results are clearly representative of the current condition of the subject. There may be a target to take the measurement 5 minutes, 4 minutes, 3 minutes or 2 minutes before the clinician visit. Thus, a target timing may be set somewhere in the range 1 minute to 5 minutes before the clinician visit.

The design of the clinician module 50 will depend upon the infrastructure in the hospital.

Figure 2 shows an example in which the clinician module 50 receives input from a camera 70. The camera is shown as not forming part of the system 20 because it may be shared between multiple bedside systems 20. Cameras are increasingly part of patient monitoring systems for applications like vital sign monitoring, bed-exit detection etc. Cameras may in this example also be used monitoring movement of clinician.

As an example, a camera placed near the central hallway of a ward, or at the entrance to a corridor, could be used to identify when the visit of a nurse or doctor to the ward is imminent. The location of the camera is in this way in advance of the location of the subject ("in advance" in terms of the movement path of the clinician when performing their patient visit rota). The camera 70 may use face-detection and person identification in order to identify the clinician being tracked. Once the camera 70 identifies an oncoming visit of the clinician, this information is relayed by the clinician module 50 to the patient care module 30. This triggers a blood pressure measurement or schedules a blood pressure measurement on time. The exact scheduling of the measurement can be configured based on the expected time for the clinician to get from detection location to the patient bed.

By scheduling the blood pressure measurement just before a clinician arrival, the so-called white coat effect is avoided, by which the presence of the clinician affects the results.

The camera-based clinician monitoring can be further augmented by discovering the routine of the clinician's rota. Patient schedule information for the clinician may for example be stored in a memory 72. This provides a further input to the clinician module 50.

Pattern recognition based on the encounter time between a clinician and the patient in the preceding days can for example be used to infer the expected visit time of the clinician for the current day, which then is used to derive or modify the schedule information. It is common practice for doctors or nurses to visit patients by a fixed schedule every day. Scheduling a blood pressure measurement according to the expected daily visit time of the clinician can be a starting point for the intelligent blood pressure measurement scheduling, where additional refinements of the scheduling are done based on available information sources as discussed above.

Some hospitals already make use of a computerized software system that keeps track of the visit of the clinician to each patient. Such a record can be populated by the hospital staff who keep track of the expected time when a doctor or other care staff is supposed to visit the patient. Thus, an expected schedule may already be available, rather than simply assuming a repeating pattern from previous days.

Thus, default schedule may be kept in memory 72 and it may be dynamically adjusted based on the timing of previous patient visits by the clinician. The default schedule indicates the order and a default timing of patient visits. This schedule may then be adjusted either in real time (i.e. while the rota is being followed that very day) or based on information from previous days, based on the actual visit times. These visit times for example depend on the variable times of the patient interactions, and any disruptions or interruptions which the clinician may encounter.

Figure 2 shows an alternative (or additional) way to track clinician location, based on an internal location tracking system 74 for example using near field communication or using a RFID system. The movement of nurses and doctors may be tracked using this location tracking system. Once it is identified that the nurse or the doctor is going to visit a patient bed, this information is relayed to the patient care module 30 which then schedules a blood pressure measurement accordingly.

The clinician module 50 then has an input for receiving location tracking information for tracking the location of the clinician. This location tracking system 74 is again shown external to the system, because it is shared between multiple bedside systems.

The blood pressure measurement system 32 is controlled by the controller 34 of the patient care module 30 either through wired or wireless connections, to trigger a measurement, as scheduled. When the timing of an upcoming visit of a clinician is determined, it may first be established if the currently available measurement was taken too long ago. Only if this is the case, then the next blood pressure measurement is scheduled such that when the clinician is at the patient bed then the available measurement results are recent.

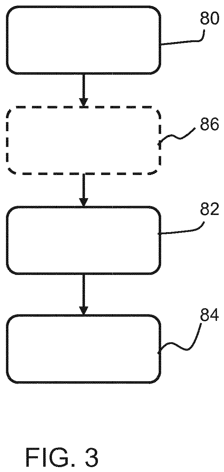

Figure 3 shows a computer-implemented method for scheduling blood pressure measurements of a subject, comprising:
in step 80, receiving patient information relating to recent patient activities and/or movements;
in step 82, receiving timing information relating to a next clinician visit to the subject; and
in step 84, processing the patient information and the timing information in order to determine a time for a next blood pressure measurement of the subject.

The method may further comprise in step 86 monitoring physiological parameters of the subject, and then step 84 also involves processing the monitored physiological parameters to determine the time for a next blood pressure measurement of the subject.

In the example above, reference is made to various separate modules. This is simply for the purpose of explaining the function of the system. In practice, there may be a set of hardware components and a single computer program/system for controlling those components. Thus, no particular division of physical components into separate modules is intended to be implied.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. The computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system (20) for scheduling blood pressure measurements of a subject, comprising:
a patient care module (30) which comprises a blood pressure measurement system;
a controller (34) communicatively coupled to a patient context module (40) arranged to generate patient information relating to recent patient activities and/or movements, and coupled to a clinician module (50) arranged to generate timing information relating to a next clinician visit, wherein
the controller (34) is adapted to process the patient information and the timing information in order to determine a time for a next blood pressure measurement of the subject.

2. The system (20) as claimed in claim 1, wherein the controller (34) is further adapted to control the blood pressure measurement system to take a blood pressure measurement at the determined time of the next blood pressure measurement of the subject.

3. The system (20) as claimed in claim 2, wherein the controller (34), based on the received timing information relating to a next clinician visit, is adapted to implement periodic blood pressure measurements when there is no imminent clinician visit.

4. The system (20) as claimed in any one of claims 1 to 3, wherein the controller (34)), based on the received timing information relating to a next clinician visit, is adapted to implement a blood pressure measurement within 5 minutes preceding an expected clinician visit.

5. The system as claimed in any one of claims 1 to 4, further comprising the patient context module (40).

6. The system (20) as claimed in claim 5, wherein the patient context module further comprises a set (46) of one or more physiological sensors for monitoring physiological parameters of the subject, and the controller (34) is further adapted to process the monitored physiological parameters to determine the time for a next blood pressure measurement of the subject.

7. The system as claimed in claim 6, wherein the physiological sensors (46) comprise one or more of a respiration rate sensor, a heart rate sensor and a temperature sensor.

8. The system as claimed in any one of claims 5 to 7, wherein the patient context module (50) generates patient information relating to one or more of:
recent nursing interventions;
medications taken by the subject; and
food consumption of the subject.

9. The system as claimed in any one of claims 5 to 8, wherein the patient context module (40) comprises a camera system (44) for tracking at least movement of the subject.

10. The system as claimed in any one of claims 1 to 9, further comprising the clinician module (50).

11. The system as claimed in claim 10, wherein the clinician module (50) comprises:
an input for receiving camera information for detecting the presence of the clinician at a location in advance of the location of the subject; and/or
an input for receiving location tracking information for tracking the location of the clinician.

12. A system as claimed in any one of claims 10 to 11, wherein the clinician module (50) receives patient schedule information for the clinician, wherein the patient schedule is dynamically adjusted based on the timing of previous patient visits by the clinician.

13. A computer-implemented method for scheduling blood pressure measurements of a subject, comprising:
(80) receiving patient information relating to recent patient activities and/or movements;
(82) receiving timing information relating to a next clinician visit to the subject; and
(84) processing the patient information and the timing information in order to determine a time for a next blood pressure measurement of the subject.

14. A method as claimed in claim 13, further comprising (86) monitoring physiological parameters of the subject, and also processing the monitored physiological parameters to determine the time for a next blood pressure measurement of the subject.

15. A computer program comprising computer program code means which is adapted, when said program is run on a computer, to implement the method of claim 13 or 14.
